# EUROPEAN PATENT SPECIFICATION

(11) **EP 0 390 921 B1**
(45) Date of publication and mention of the grant of the patent: **11.10.1995**
(21) Application number: 89900116.8
(22) Date of filing: 01.12.1988
(51) Int. Cl.: D06C 29/00, D02G 3/04, A61L 27/00

(54) **KNIT OR WOVEN TEXTILE, PROCESS FOR ITS PRODUCTION AND BLOOD VESSEL REPAIRING SHEET AND ARTIFICIAL BLOOD VESSEL MADE THEREFROM**
GEWIRKTES ODER GEWOBENES TEXTILMATERIAL, VERFAHREN ZUR HERSTELLUNG UND BLUTGEFÄSSREPARATURBLATT SOWIE KUNSTBLUTGEFÄSS DARAUS
TEXTILE TRICOTE OU TISSE, PROCEDE DE PRODUCTION ET FEUILLE DE RECONSTITUTION DE VAISSEAUX SANGUINS ET VAISSEAU SANGUIN ARTIFICIEL AINSI REALISE

(30) Priority: 08.12.1987 JP 310662/87
(43) Date of publication of application: 10.10.1990
(73) Proprietor: TERUMO KABUSHIKI KAISHA, Tokyo 151 (JP)
(72) Inventor: MATSUMOTO, Atsushi Terumo Kabushiki Kaisha, Shizuoka-ken 417 (JP); IKAWA, Satoshi Terumo Kabushiki Kaisha, Shizuoka-ken 417 (JP); KATAKURA, Takeo Terumo Kabushiki Kaisha, Shizuoka-ken 417 (JP); TANABE, Susumu Terumo Kabushiki Kaisha, Fuji-shi Shizuoka-ken 417 (JP); MORI, Yuichi, Kawasaki-shi Kanagawa-ken 214 (JP)
(74) Representative: Casalonga, Axel
(86) International application number: JP8801217
(87) International publication number: WO8905371

(56) References cited:
- WO-A-87/04197
- FR-A- 2 584 744
- JP-A- 5 170 390
- JP-A- 5 524 095
- JP-A-59 218 158
- US-A- 3 986 828
- US-A- 4 191 218

## Description

This invention relates to a blood vessel repair or restorative material that is not affected in porosity, flexibility or suturability and that is not liable to be raveled at cut ends, and the method for producing the material.

At present, a tubular artificial blood vessel employed in a larger blood vessel having an inside diameter of not less than 5 mm or a blood vessel repair or restorative material in the form of a sheet-like patch is mainly prepared from a knitted or woven fabric of polyester or polytetrafluoroethylene fibers or from previously expanded porous polytetrafluoroethylene graft. Above all, the knitted fabric is formed by spinning with polyester type bulky filament formed by monofilaments having the diameter of 20 µm (monofilaments presenting meandering curves) and weaving or knitting this filament by plain weave or knitting.

On the other hand, an artificial blood vessel or a patch for blood vessel presenting the different degree of porosity (water permeability), shape or flexibility is frequently used in dependence upon the occasional transplant position or status of the patients. Thus the doctor usually selects a suitable restorative material and cuts it to suitable size to meet occasional requirements. At this time, yarn raveling or waste of the knitted fabric tends to be produced at the cut end or edge, whilst , since the fabric is anastomosed with the living tissue at this cut end or edge, a sufficient anastomotic strength cannot be produced, such that the blood tends to leak out thereat or the perimetral portion of the repaired position tends to be contaminated.

Among the features of the knitted fabric required of the blood restorative material are a sufficient curability and the blood leakage preventive effect at the initial stage of transplantation. For improving the curability, it is necessary to increase water permeability; however, the risk of blood leakage becomes that high with increase in water permeability. For this reason, woven fabrics of lower water permeability have come into use more frequently. However, the woven fabric is also beset with a shortcoming that it is more liable to yarn raveling or waste than the knitted fabric.

US-A-4 191 218 discloses fabric for heart valve and vascular prostheses comprising multifilament synthetic yarns which are mutually separated by interstices in the woven pattern. The fabric is compressively shrunk in two different directions to form crimps in the yarns and the crimps are heat set to lie in planes generally parallel with the fabric. The yarns are bloomed in the interstices to form spaces of varying sizes and orientations between the filaments.

WO-A-87 04197 discloses a fabric wherein the yarn constituting the fabric comprises a monofilament having a melting point significantly lower than the reminder of the monofilaments and wherein the monofilament of lower melting point is present over the full length of the yarn. As a consequence, the monofilament of low melting point will melt over its full length resulting in the physical properties of the yarn being modified over its full length by impregnation of the molten filament and the yarns being bonded at all contact points.

It is an object of the present invention to overcome this inconvenience of the prior art and to provide a knitted or woven fabric which is not affected in porosity or flexibility and which is not liable when cut to yarn raveling or waste, the method for producing the fabric and the blood restorative sheet and the artificial blood vessel formed by such fabric.

The present invention provides a blood vessel restorative material characterized in that it is formed as a sheet from a woven or knitted fabric, said knitted fabric or woven fabric having predetermined ones of contact points between yarns affixed with an adhesive, there being substantially no adhesive in voids exclusive of contact points of the yarns, the fabric exhibiting moderate flexibility and water permeability.

The present invention also provides an artificial blood vessel formed as a tube from a woven or knitted fabric as defined above.

The present invention also provides a method for producing a woven fabric or a knitted fabric for use as a blood vessel restorative material as defined above characterized in that it comprises:
- applying a heat-sensitive adhesive at a plurality of predetermined spaced apart positions onto flexible yarns;
- weaving or knitting said flexible yarns having applied thereon heat-sensitive adhesive at said plurality of predetermined spaced apart positions so that said heat-sensitive adhesive is located on at least a part of the contact points of said yarns upon knitting or weaving of said yarns;
- heat-melting said fabric to melt said heat-sensitive adhesive to cause the yarns of the fabric to adhere to each other at said contact points;
- allowing said heated fabric to cool.

Fig. 1 is a partial diagrammatic view of a knitted fabric according to the present invention.

Fig. 2 is a diagrammatic view showing the state in which the knitted fabric shown in Fig. 1 is cut partially.

Fig. 3 is a diagrammatic view showing an example of means for applying an adhesive to the yarn.

The present invention will be explained in more detail hereinbelow.

There are a sheet-like patch or a tubular artificial blood vessel, as the blood vessel restorative material, as mentioned above. These are generally constituted by knitted fabrics since porosity (water permeability) and flexibility are among the requirements As a result of our researches towards eliminating the above described drawbacks of the blood vessel restorative material of the prior art, the present inventors have succeeded in evolving a knitted or woven fabric which is not liable when cut to formation of yarn raveling or waste and which is endowed with porosity (water permeability) and flexibility, the method for producing the fabric and the blood repair or restorative material formed by the fabric.

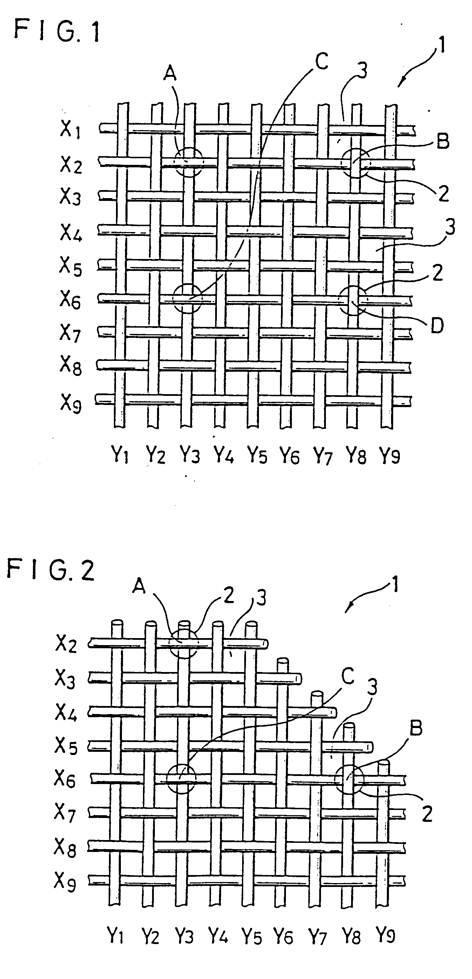

Fig.1 is a plan view showing an embodiment of using the plain weave among the woven and knitted fabrics of the present invention. The knitted or woven fabrics designate the woven fabrics and the knitted fabrics. Although the following description is made with reference to this woven fabric as a representative example, the present invention also applies to the knitted fabric as well.

As shown in Fig.1, a fabric 1 is woven from warp yarn yᵢ, wherein ᵢ is 1 to 9 in Fig.1, and weft yarn xᵢ, wherein ᵢ is 1 to 9 in Fig.1. Part of the points of intersection or contact between the warp yarn and the weft yarn are bonded at predetermined positions with a heat meltable adhesive 2 applied to each yarn at predetermined intervals. In the woven fabric shown partially in Fig.1, such bonding is made at contact points A (x2, y3), B (x2, y7), C (x6, y3) and D (x6, y2). However, most of the adhesive 2 is not admitted into the voids 3 of the woven fabric 1 and the voids 3 are not closed by the adhesive 2.

It is assumed that the fabric shown in Fig.l is cut as shown in Fig.2. When it is also assumed that the contact points of the fabric 1 are not bonded with the adhesive 2, the weft yarn x2 tends to be dislocated from the woven fabric and is turned into yarn raveling or waste. With the weft yarn x2 detached from the woven fabric, the weft yarn x3 also tends to be raveled.

Conversely, when the warp yarn y3 and the weft yarn x2 are bonded to each other at the point of contact A with the adhesive 2 according to the present invention, raveling of the weft yarn x2 is stopped at the bonding point A between the warp yarn y3 and the weft yarn x2 without proceeding any further. Thus the weft yarn x3 is also prevented from raveling.

On the other hand, if attention is directed solely to raveling, it may be possible to resort to a method consisting in dipping the fabric in an adhesive dissolved in a solvent to coat the fabric with the adhesive on the whole, or to a method consisting in partially printing the woven fabric with an adhesive. However, with these methods, the raw yarns will adhere to each other and most of all contact points between the yarns will adhere to each other, with the result that flexibility required of the blood vessel restorative material is lost and since the adhesive is admitted to voids of the woven fabric, water permeability is also lowered, so that it becomes difficult to turn the material into a satisfactory organ.

Conversely, in the present invention, flexibility and water permeability are not affected substantially because there are only few connecting points between the yarns adhered by the adhesive and almost all the adhesive is not admitted to the voids of the woven fabric.

On the other hand, the blood vessel restorative material is a fabric knitted or woven so closely that, in the case of the plain weave, adjacent warp or weft yarns contact each other. Therefore, when the adhesive is applied partially to the woven or knitted fabric after weaving or knitting, the major portion of the yarn is bonded together by the adhesive seeping into the yarn with the result that the fabric is affected in flexibility. On the other hand, the effect of preventing raveling is lowered because some yarns are not affixed with the adhesive.

According to the present invention, the effect of preventing raveling is significant because the adhesive is applied to the yarns at predetermined intervals before weaving or knitting, so that the yarn remaining quite unaffixed to the adjacent yarns may be eliminated even in the case of plain weave.

As the starting material of the yarn employed in the present invention, polyester or polytetrafluoroethylene may be employed. However, from the viewpoint of biocompatibility and compatibility with the adhesive, polyester is most preferred.

As the adhesive to be applied to the yarns, any adhesive having the melting point not higher than the melting point of the yarn may be employed, such as, for example, polyurethane, polystyrene, polyolefin or gelatin.

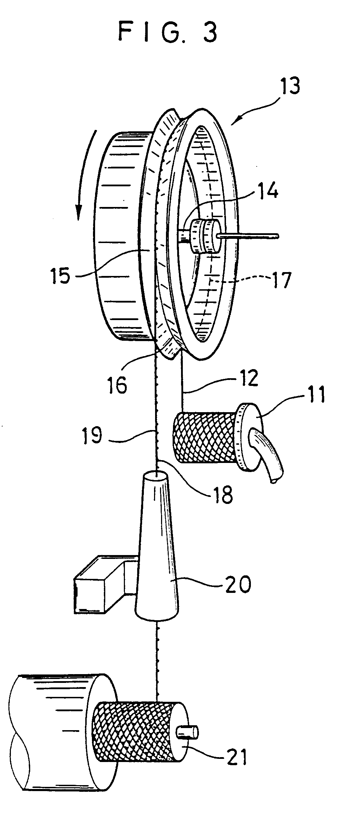

Fig.3 shows, only by way of an illustrative example, means for applying an adhesive to the yarn at predetermined intervals.

Turning to Fig.3, an adhesive is applied at a printing drum 13 to a raw yarn 12 reeled out from a spool 11 The printing drum 13 has a rotary drum 15 revolving about a rotational shaft 14 which is provided with a groove 16 in which the raw yarn 12 is laid. A number of orifices 17 are formed at predetermined intervals in the groove 16.

The adhesive seeps out of these orifices 17 so that the adhesive 18 is applied at predetermined intervals on the raw yarn 12.

The treated yarn 19 to which the adhesive 18 is applied as described above is dried in a dryer 20 before being taken up on a take-up spool 21. In this manner, the yarn to which the adhesive has been applied at predetermined intervals for preparing the knitted or woven fabric of the present invention is obtained.

The yarn obtained by the method shown for example in Fig.3 may then be woven or knitted in a conventional manner to produce the woven or knitted fabric according to the present invention.

This woven or knitted fabric is then heat-treated to melt only the adhesive to cause part of the points of contact or intersection between the warp and weft yarns of the fabric with the adhesive. This completes the woven or knitted fabric of the present invention.

The woven or knitted fabric thus formed is used mainly as the blood vessel restorative material which may be in the form of a sheet. Such a sheet-like patch may be cut to a required size to repair e.g. a hole punched in the blood vessel.

The blood vessel restorative material may also be knitted or woven into a tube. The resulting tubular fabric is used as an artificial blood vessel in substitution for a portion of an injured blood vessel.

In the woven or knitted fabric of the present invention, the interposed adhesive is applied only in the vicinity of a part of the contact points of the yarns for affixture, while virtually no adhesive is applied to the voids between the yarns, Thus the fabric is not affected in flexibility, as compared with the woven or knitted fabric produced from the yarns not treated with the adhesive, while exhibiting moderate flexibility and sufficient porosity or water permeability, so that it may be employed satisfactorily as the blood vessel restorative material.

The present invention will be explained in more detail with reference to an illustrative example thereof.

Using a rotary press shown in Fig.3, polyurethane dissolved in a solvent was applied to a 5 denier polyester fiber, at intervals of 2 mm , each to a width of 100 µm.

The thus produced yarn was woven by plain weave and processed into a patch for blood vessel which was then heated to 185°C. The patch for blood vessel produced is not affected substantially in flexibility, as compared with the patch produced from the yarn not treated with the adhesive, while remaining substantially free from yarn raveling or waste when cut with scissors. Although water permeability is lowered by about 5 %, such a minor decrease in water permeability does not raise practically any problem.

In the woven or knitted fabric of the present invention, the interposed adhesive is applied only in the vicinity of a part of the contact points of the yarns for affixture, while virtually no adhesive is applied to the voids between the yarns, Thus the fabric is not affected in flexibility, as compared with the woven or knitted fabric produced from the yarns not treated with the adhesive, while exhibiting moderate flexibility and sufficient porosity or water permeability, so that it may be employed satisfactorily as the blood vessel restorative material.

Such woven or knitted fabric may be produced easily and inexpensively by a method consisting in applying an adhesive to the raw yarns at predetermined intervals, weaving or knitting the yarns to form a fabric and heating the fabric to melt the adhesive to cause the yarns of the fabric to adhere to each other at a part of the contact points between the yarns.

## Claims

1. A blood vessel restorative material characterized in that it is formed as a sheet from a woven or knitted fabric (1), said knitted fabric or woven fabric having predetermined ones of contact points (A,B,C,D) between yarns (xᵢ,yᵢ,12) affixed with an adhesive (2,18), there being substantially no adhesive in voids exclusive of contact points of the yarns, the fabric exhibiting moderate flexibility and water permeability.

2. A blood vessel restorative material according to claim 1 wherein said fabric (1) is a flexible, water permeable, fray resistant fabric.

3. An artificial blood vessel formed as a tube from the fabric (1) according to claim 1 or 2.

4. A method for producing a woven fabric or a knitted fabric (1) for use as a blood vessel restorative material according to claim 1 or 2 characterized in that it comprises:
- applying a heat-sensitive adhesive (2,18) at a plurality of predetermined spaced apart positions onto flexible yarns (xᵢ,yᵢ,12);
- weaving or knitting said flexible yarns having applied thereon heat-sensitive adhesive at said plurality of predetermined spaced apart positions (A,B,C,D) so that said heat-sensitive adhesive is located on at least a part of the contact points of said yarns upon knitting or weaving of said yarns;
- heating said fabric to melt said heat-sensitive adhesive to cause the yarns of the fabric to adhere to each other at said contact points provided with said heat-sensitive adhesive; and
- allowing said heated fabric to cool.

5. The process of claim 4 wherein said heat-sensitive adhesive (2,18) has a lower melting point than that of the yarns (xᵢ,yᵢ,12).

## Patentansprüche

1. Blutgefäßwiederherstellungsmaterial, dadurch gekennzeichnet, daß es als Bogen aus gewobenem oder gewirktem Gewebe (1) gebildet ist, wobei das gewirkte Gewebe oder gewobene Gewebe vorbestimmte Berührungspunkte (A;B;C;D) zwischen Garnen (xᵢ,yᵢ) hat, welche mit einem Haftmittel (2,18) befestigt sind, wobei in den Hohlräumen, ausgenommen den Berührungspunkten der Garne, im wesentlichen kein Haftmittel vorhanden ist, wobei das Gewebe eine mäßige Flexibilität und Wasserdurchlässigkeit aufweist.

2. Blutgefäßwiederherstellungsmaterial nach Anspruch 1, wobei das Gewebe (1) ein flexibles, wasserdurchlässiges, gegen Ausfransen widerstandsfähiges Gewebe ist.

3. Künstliches Blutgefäß aus einem Rohr aus dem Gewebe (1) nach Anspruch 1 oder 2.

4. Verfahren zur Herstellung eines gewobenen Gewebes oder eines gewirkten Gewebes (1) für die Anwendung als Blutgefäßwiederherstellungsmaterial nach Anspruch 1 oder 2, dadurch gekennzeichnet, daß es folgendes aufweist:
- Auftragen eines wärmeempfindlichen Haftmittels (2,18) an einer Vielzahl von vorbestimmt beabstandeten Positionen auf flexiblen Garnen (xᵢ,yᵢ,12);
- Weben oder Wirken der flexiblen Garne, auf welche an der Vielzahl von vorbestimmt beabstandeten Positionen (A,B,C,D) ein wärmeempfindliches Haftmittel auftragen ist, so daß das wärmeempfindliche Haftmittel auf zumindest einem Teil der Berührungspunkte der Garne nach dem Wirken oder Weben der Garne angeordnet ist;
- Erwärmen des Gewebes, um das wärmeempfindliche Haftmittel zu schmelzen, damit die Garne des Gewebes an den Berührungspunkten aneinander haften, die mit dem wärmeempfindlichen Haftmittel versehen sind; und
- Abkühlen lassen des erwärmten Haftmittels.

5. Verfahren nach Anspruch 4, wobei das wärmeempfindliche Haftmittel (2,18) einen niedrigeren Schmelzpunkt als der von den Garnen (xᵢ,yᵢ,12) hat.

## Revendications

1. Tissu de restauration de vaisseau sanguin, caractérisé en ce qu'il est formé en feuille à partir d'une étoffe tissée ou tricotée (1), ladite étoffe tricotée ou ladite étoffe tissée présentant certains points de contact prédéterminés (A, B, C, D) entre les fils (xi, yi, 12) fixés par une colle (2, 18) pratiquement aucune colle n'étant présente dans les vides à l'exclusion des points de contact entre les fils, l'étoffe présentant une flexibilité et une perméabilité à l'eau modérées.

2. Tissu de restauration de vaisseau sanguin selon la revendication 1, dans lequel ladite étoffe (1) est une étoffe flexible, perméable à l'eau, résistant à l'effilochement.

3. Vaisseau sanguin artificiel formé en tant que tube à partir de l'étoffe (1) selon la revendication 1 ou 2.

4. Procédé de production d'une étoffe tissée ou d'une étoffe tricotée (1) à utiliser comme tissu de restauration de vaisseau sanguin selon la revendicaiton 1 ou 2, caractérisé en ce qu'il comprend les étapes consistant à :
- appliquer une colle thermosensible (2, 18) en une pluralité de positions prédéterminées distantes les unes des autres sur des fils flexibles (xi, yi, 12);
- tisser ou tricoter lesdits fils flexibles sur lesquels a été appliquée la colle thermosensible en ladite pluralité de positions prédéterminées distantes les unes des autres (A, B, C, D) de façon que ladite colle thermosensible soit localisée en au moins une partie des points de contact desdits fils lors du tissage ou du tricotage desdits fils;
- chauffer ladite étoffe pour faire fondre ladite colle thermosensible pour coller les fils de l'étoffe les uns aux autres auxdits points de contact dotés de ladite colle thermosensible et
- laisser refroidir ladite étoffe chauffée.

5. Procédé selon la revendication 4, dans lequel ladite colle thermosensible (2, 18) présente un point de fusion inférieur à celui des fils (xi, yi, 12).
